Europäisches Patentamt

European Patent Office

Office européen des brevets

(19)

(11) Publication number: **0 439 214 A1**

# EUROPEAN PATENT APPLICATION

(12)

(21) Application number: **91200049.4**

(22) Date of filing: **12.01.91**

(51) Int. Cl.5: **G01N 33/569**, G01N 33/543, G01N 33/546

(30) Priority: **22.01.90 US 468034**

(43) Date of publication of application:
**31.07.91 Bulletin 91/31**

(84) Designated Contracting States:
**CH DE FR GB IT LI NL SE**

(71) Applicant: **EASTMAN KODAK COMPANY**
**343 State Street**
**Rochester, NY 14650-0221(US)**

(72) Inventor: **Snyder, Brian Anthony, c/o**
**EASTMAN KODAK COMPANY**
**Patent Department, 343 State Street**
**Rochester, New York 14650-2201(US)**
Inventor: **Zambon, Joseph James, c/o**
**EASTMAN KODAK COMPANY**
**Patent Department, 343 State Street**
**Rochester, New York 14650-2201(US)**
Inventor: **Reynolds, Homer Stanley, c/o**
**EASTMAN KODAK COMPANY**
**Patent Department, 343 State Street**
**Rochester, New York 14650-2201(US)**

(74) Representative: **Nunney, Ronald Frederick**
**Adolphe et al**
**Kodak Limited Patent Department Headstone**
**Drive**
**Harrow Middlesex HA1 4TY(GB)**

(54) **Screening assay for microorganisms associated with periodontal diseases, article and kit useful therein.**

(57) A screening method is provided for indiscriminate detection of microorganisms associated with various periodontal diseases, such as any of the microorganisms Actinobacillus actinomycetemcomitans, Bacteroides gingivalis and Bacteroides intermedius. In this screening method, a specimen suspected of containing antigens from one or more of these microorganisms is contacted with a microporous membrane on which immunological complexes are formed with the appropriate antibodies. In order for screening to occur, antibodies to at least one antigen from each microorganism is used. Any complexes formed on the membrane can be detected using appropriate labels and detection means to indicate the presence of at least one of the microorganisms. This method does not discriminate among the microorganisms. Also provided is a water-insoluble article and kit useful in practicing the screening method.

EP 0 439 214 A1

## SCREENING ASSAY FOR MICROORGANISMS ASSOCIATED WITH PERIODONTAL DISEASES, ARTICLE AND KIT USEFUL THEREIN

This invention relates to a method for screening a biological specimen for the presence of any microorganisms associated with periodontal diseases, such as the microorganisms Actinobacillus actinomycetemcomitans, Bacteroides gingivalis and Bacteroides intermedius. It also relates to a water-insoluble article and kit useful in the screening method. This invention is useful in dental research and health care.

There is a continuing need in medical, dental and veterinary practices, and in research and diagnostic procedures, for rapid and accurate detection or quantification of biological or chemical substances present in biological fluids or specimens. For example, the presence of various microorganisms in human and animal tissues, fluids or cells is very important for diagnosis and effective treatment of diseases.

Specific microorganisms have been implicated as indicators for a number of periodontal diseases in humans and animals, such as gingivitis and periodontitis. The significance of such diseases is growing in the human population, especially as people live longer, and prevention thereof is becoming of considerable medical and commercial importance. In addition, the proper care of animals (including dental care) is a growing concern in our culture.

Detection of microorganisms associated with periodontal diseases has been accomplished using culture techniques (which are generally slow), DNA probes and a number of immunological procedures, such as agglutination assays, enzyme linked immunosorbent assays (ELISA), immunofluorescence and immunoprecipitation. The immunological procedures use immunological reactions of an antigenic site of the microorganism (which may be on a component extracted therefrom) with a corresponding antibody specific thereto. The resulting immunological reaction complex can then be detected in a number of ways.

Actinobacillus actinomycetemcomitans (identified herein as A. actinomycetemcomitans) is an oral gram-negative facultative organism which is closely related to the Haemophilus group of organisms. This organism can cause severe human infections, including bacterial endocarditis, thyroid gland abscess, urinary tract infection, brain abscess and vertebral osteomyelitis. It has been strongly implicated in the pathogenesis of certain types of periodontal disease, particularly in localized juvenile periodontitis and also in some forms of adult periodontitis. Human isolates of A. actinomycetemcomitans have been divided into three major serogroups designated as serotypes A, B and C.

The black-pigmented Bacteroides species are gram negative, anaerobic rods which are common in the etiology of various periodontal diseases, odontogenic abscesses and endodontic lesions. Such species include B. intermedius and B. gingivalis. B. gingivalis is also known in the art as Porphyromonas gingivalis or Bacteroides(Porphyromonas) gingivalis. As defined herein, periodontal disease refers to a wide variety of diseases in humans and animals which occur in the periodontal area of the oral cavity, and includes diseases affecting the connective tissue, loss of alveolar bone as well as the gingiva. It includes diseases that may occur at different times of human life, such as in infancy, youth, pregnancy or old age. B. intermedius and B. gingivalis have been associated with certain periodontal diseases.

Clinical assays specific to the bacteria noted above in gingival crevicular fluid and subgingival dental plaque are useful in the diagnosis of periodontal disease, in evaluating the options for and progress of periodontal treatment, and in determining the status of the patient at later dental examinations. The standard culture techniques used to identify such organisms are time consuming, expensive and require a high level of operator expertise. Such tests also may lack sufficient sensitivity for detection of low levels of organisms due to strict anaerobic conditions required during transport.

The various immunoassays noted above have been developed and used with some success. Particularly useful are radioimmunoprecipitation assays and ELISA tests. US-A-4,741,999 describes monoclonal antibodies to antigens of A. actinomycetemcomitans and their use in ELISA assays. While these tests provide improvements over the standard culture techniques, further improvements are needed to increase assay sensitivity to the desired organism while at the same time minimizing cross-reactivity with other organisms which are similar, such as those in the Haemophilus group.

Monoclonal and polyclonal antibodies to various Bacteroides species have been prepared for similar assays [see for example, Nakazawa and others, Infect. Immun., 56(6), pp. 1647-1651, 1988, and Zambon and others, J.Periodon., 56(Supp), pp. 32-40, 1985].

Generally, the assays of the art have been slow, tedious and directed to a single organism. In many cases, the assays are highly cross-reactive with related species, and thus have limited usefulness. Since a number of microorganisms, including A. actinomycetemcomitans, B. gingivalis and B. intermedius, have been implicated in various periodontal diseases, it would be highly useful to a practitioner to have a means for detecting any of such microorganisms in a screening test so it could be determined if a patient needed periodontal treatment. Other microorganisms may be implicated in the future as causing such diseases. Such a test would need to be inexpensive and simple to use for it to find extensive application in a practitioner's office. None of the assays described in the art meet these important considerations.

The problems noted above with known assays have been overcome with a method for the simultaneous, indiscriminate detection of any microorganism associated with periodontal disease, comprising:

A. contacting a specimen suspected of containing one or more antigens extracted from one or more microorganisms associated with periodontal disease with a microporous substrate, and forming on the substrate one or more immunological complexes by contacting the one or more antigens simultaneously with one or more antibodies directed to a plurality of microorganisms associated with periodontal disease, and

B. detecting the presence of the one or more complexes on the substrate as an indication of the presence of at least one of the antigens in the specimen.

This invention also provides a water-insoluble article comprising a microporous substrate having first and second opposing outer surfaces,

the article characterized wherein it has randomly affixed to at least one of the surfaces, one or more antibodies directed to a plurality of microorganisms associated with periodontal diseases, the one or more antibodies being substantially all on the first or second surface.

Moreover, a test kit for the simultaneous, indiscriminate detection of any microorganism associated with periodontal disease comprises:

a. a water-insoluble article comprising a microporous substrate having first and second opposing outer surfaces, and one or more unlabeled antibodies randomly affixed to at least one of the surfaces, and

b. detectably labeled antibodies having the same reactivity as the affixed antibodies, the labeled antibodies being packaged separately or in admixture,

the kit characterized wherein said unlabeled antibodies are directed to a plurality of microorganisms associated with periodontal disease, the antibodies being substantially all on the first or second surface.

The method of this invention is an effective, rapid and inexpensive means for screening a biological specimen obtained from a patient for any of the microorganisms associated with periodontal diseases. In particular, the screening test is useful for detecting any of A. actinomycetemcomitans, B. gingivalis and B. intermedius. Prior to this time, each microorganism was detected by tedious culture techniques individually, or with more expensive and less sensitive immunological methods known in the art.

This invention is convenient for a practitioner who may want to screen a dental plaque or gingival crevicular fluid sample in the office while dental work continues on the patient. Thus, before the patient leaves, the practitioner knows if the patient should be treated for the presence of any of the microorganisms, often before there are any noticeable signs of disease. Such preventative care is highly desired today for many reasons. The patient and practitioner also need not wait a long time for the results of the screening when using this invention.

These long awaited advantages are achieved by using an immunological method which contacts the specimen with one or more antibodies which are reactive to a number of microorganisms substantially simultaneously. Thus, if any one of the microorganisms (or antigenic components thereof) is present, antigenic sites therefrom will be complexed with the appropriate antibodies and the resulting complexes can be appropriately detected. The complexation occurs on a microporous substrate so that uncomplexed materials are quickly removed, and any remaining complex is retained for detection.

The present invention can be used to rapidly and sensitively screen for the presence of one or more microorganisms associated with periodontal diseases. Such microorganisms, include but are not limited to, A. actinomycetemcomitans, B. gingivalis and B. intermedius. It is quite likely that other microorganisms have been or will be implicated in periodontal diseases in the future. It is not important in this method as to which microorganism may be present, or how many. Any serotype of any of the microorganisms may be present. This method is generally qualitative although the amount of complex observed in the detection technique may indicate a relative amount of the microorganisms. While the antigens detected with the method (lipopolysaccharides, capsule antigens or membrane proteins) may be part of intact cells, normally, they are extracted in a suitable manner from the cells present in a biological specimen. Such specimens include, but are not limited to, saliva or mucous from the throat or mouth, human or animal tissue extracts, dental plaque and gingival crevicular fluid. Generally, the organism is detected in dental plaque, saliva, or gingival crevicular fluid.

As noted above, this is primarily a screening method, and it may be included as an initial assay in a series of assays, the later assays being used for differentiating microorganism species or serotypes, or for monitoring the progress of treatment. Such later assays can be any of those already known in the art, such

3

as direct binding, ELISA or sandwich assays.

Antigen extraction from any of the microorganisms of interest is suitably accomplished using physical or chemical means, such as by use of a detergent (such as sodium dodecyl sulfate, sodium decyl sulfate or sodium deoxycholate) using standard procedures, as described, for example, in US-A-4,741,999, osmotic shock [see for example, Dirienzo and others, Infect. & Immun., 47(1), pp. 31-36, 1985], or sonic means [see for example, Zambon and others, Infect. & Immun., 41(1), pp. 19-27, 1983].

If desired, the antigenic material can be removed from the original specimen, or the original specimen can be suitably diluted with water or a suitable buffer or filtered in order to remove extraneous matter and facilitate complexation of antigen and antibody in the assay.

Antibodies used in the practice of this invention can be monoclonal or polyclonal. Monoclonal antibodies can be prepared using standard procedures, such as those described in US-A-4,741,999 (noted above). Polyclonal antibodies can also be produced using standard procedures, such as described, for example by Zambon and others, Infect. & Immun., 41(1), pp. 19-27 (1983). Generally, a mammal (such as a rabbit) is immunized one or more times with a suitable quantity of an antigenic component or whole bacterial cells of the organism. After a suitable time, when the titer is acceptable, antisera is recovered from the mammal. Antibodies can be removed from the antisera and purified if desired using known procedures and stored in frozen buffered solutions until used. Further details of such procedures are well known in the art.

Generally, once the antibodies are produced in a suitable manner, they can be isolated or stored for used in buffered solutions containing one or more suitable buffers and a preservative if desired. Preferably, they are stored in frozen form. In the buffered solutions, the pH is generally maintained at from 6 to 9, and preferably at from 6.5 to 8.5 using suitable buffers which are readily apparent to one skilled in the art, including tricine, bicine, phosphate buffered saline solution, tris(hydroxymethyl)aminomethane, N-tris (hydroxymethyl)methyl-2-aminoethanesulfonic acid.

The antibodies for each antigen can be stored and supplied separately in the assay or kit, or they can be mixed together for storage prior to usage. Since the specimen is being screened for a plurality of microorganisms, generally at least two separate antibodies are used, and preferably more are used. In an ideal situation, a separate antibody responsive to each microorganism believed to be present is utilized. However, it is possible that a single antibody may be reactive with more than one microorganism.

In a preferred embodiment, in screening for A. actinomycetemcomitans, B. gingivalis and B. intermedius, at least three antibodies are generally used, although some antibodies will react with both Bacteroides antigens. It the latter case, only two separate antibodies would be used in the screening assay. Because each of the noted microorganisms exists in at least three serotypes, it is possible that more than three antibodies would be needed in the assay. One skilled in the art would be able to readily determine the number of antibodies to use, and the relative concentrations of each for adequate screening.

The screening assay of this invention is carried out by forming an immunological complex of the extracted antigens and the antibodies on a microporous substrate. Contact of the antigens and all of the antibodies is done substantially simultaneously and indiscriminately so that complexation between the individual antigens and their respective antibodies occurs at about the same time. There may be a brief period of incubation at room temperature, or at a higher temperature, to allow sufficient complexation for detection.

The microporous substrate generally has first and second outer surfaces, and is water-insoluble and inert to any chemical or biological reactions. It is generally composed of one or more natural or synthetic substances which have sufficient integrity for reagents to react or be affixed thereto without loss of form or function. It is porous enough for filtration needed to remove substantially all uncomplexed materials from the complexes formed thereon. Useful materials include, but are not limited to, porous natural or synthetic polymers, sintered glass, membranes of glass or polymeric films or fibers, ceramic materials, cellulosic materials and particulate structures composed of beads bound together with adhesives or binder materials. The substrates are generally flat, but some irregularities in the surfaces are acceptable, as well as some curvature, if it is desired. One skilled in the art would be able to identify other useful materials which are commercially available or prepared using known techniques. Particularly useful materials are treated or untreated polyamide microporous membranes, such as those commercially available from Pall Corp.

The microporous substrate generally has an average pore size of from 0.5 to 10 $\mu$meters, although smaller or larger pores would be acceptable as long as the complexes formed remain on the outer surface, and drainage is not undesirably long.

The substrate is designed such that it has sufficient porosity to quickly drain away fluid and uncomplexed materials encountered during an assay. Such uncomplexed materials include uncomplexed antigen, antibodies, cellular debris and other nonparticulate extraneous matter from a biological specimen. Therefore, the pore size of the substrate must be such that the noted materials will pass through it.

Moreover, while some of the reagents may become entrapped within the pores, the pore size generally will not accommodate a significant portion of the reagents used in the assay. It is highly preferred that substantially all of the complex be on the substrate outer surface. Thus, less than 1%, by weight, of any antibodies (immobilized or not) are entrapped by the microporous substrate (such as a membrane). By "entrapped" is meant that the entire reagent is within a pore of the membrane.

If desired, the microporous substrate can be coated with proteins (such as casein or succinylated casein), as known in the art to reduce nonspecific interactions, or by surfactants to promote rapid filtration, or other optional materials which may facilitate the assay.

The substrate has two surfaces which oppose each other. Generally, the surfaces are the upper and lower surfaces of a membrane. This is to distinguish the material from multilayer analytical elements known in the art which have one or more porous adjacent layers.

The microporous substrate can be hand-held in the assay to provide sites thereon for complexation and filtration of the specimen into a separate container. However, preferably, the substrate is disposed or mounted in a water-insoluble article having a water-insoluble frame or structure for holding the substrate. Such articles are often identified in the art as "test devices", and many forms of such devices are known in the art. Particularly useful devices are those marketed as part of Surecell™ test kits (Eastman Kodak Co.) for detection of antigenic materials. Such devices are described in EP-A-0 308 231 and EP-A-0 321 261.

More specifically, the preferred test device comprises a water-insoluble shell having one or more test wells therein, each of which can accommodate a sample of a biological specimen and appropriate reagents. The shell can be prepared from any useful water-insoluble material such as glass, polymeric materials, ceramics, fibrous materials, cellulosic materials and other materials known in the art.

In a preferred embodiment, the test device has three test wells designed for providing a specimen screening result and positive and negative control results. Each test well has a microporous substrate mounted therein. Other variations of useful test devices would be within the purview of a worker of ordinary skill in the art.

The assay can be carried out in one or more discrete zones of the substrate, each zone representing less than the total area of its surface. Alternatively, the entire surface can be used. Such zones may be provided by affixing the antibodies in some fashion (as described below) to a region of the surface, while leaving surrounding area free of antibodies. Preferably, the discrete zone is a round spot.

The method of this invention can be carried out in any fashion in which the antibodies are contacted with the antigens on the microporous substrate so that one or more immunological complexes are formed between the antibodies and the respective antigens. In one embodiment, this contact is carried out by contacting the antigens with the microporous substrate which has the antibodies immobilized thereon. Contact can be accomplished in any suitable manner, but preferably, the specimen containing antigens is dropped or poured onto the substrate, which may be in a disposable test device.

Any complex formed thereby is then detected in a suitable manner as an indication of the presence of the antigen (and hence, one of the microorganisms) in the specimen. Detection procedures will depend upon the type of assay being carried out and the type of label used, if any, and will include the use of appropriate reagents and equipment.

In one embodiment, the antigens in the specimen can be directly bound (or captured) on the microporous substrate in what is called a "direct binding" assay. Once attached to the substrate, they are contacted with the antibodies to form one or more immunological complexes bound to the support. This complex can be detected if the antibodies are labeled (such as with an enzyme or radioisotope), or if unlabeled, labeled anti-antibodies directed to the first antibodies can be added to form one or more detectable antigen-antibody-antibody complexes on the support. In either case, detection is possible using a composition which provides a dye in the presence of the enzyme and its substrate. Other labels can be used, however, including avidin, biotin, or fluorescent, phosphorescent or chemiluminescent moieties attached to the antibodies.

In a preferred embodiment, the method involves the complexation of antigen with two antibodies, one being insolubilized or insolubilizable, and the other set being suitably labeled for detection. Antibodies which are insolubilized or insolubilizable in some manner, such as by attachment directly to the microporous substrate, but preferably by attachment (covalent or absorption) to polymeric particles, are described below. Complexation with the labeled and unlabeled (insoluble) antibodies can occur in sequence, or simultaneously with binding to or contact with a microporous substrate. This is generally known in the art as an immunometric or "sandwich" assay.

For instance, the extracted antigens can be contacted with immobilized antibodies on the microporous substrate, followed by contact with corresponding water-soluble labeled antibodies. These labeled antibodies have the same "reactivity" as the insoluble antibodies, that is they react with the same antigens.

Preferred labels are enzymes such as peroxidase, alkaline phosphatase, glucose oxidase, urease or $\beta$-galactosidase, but other labels (as noted above) are also useful. Detection of the sandwich complex on the substrate can be achieved using appropriate substrates and dye-providing reagents.

Immobilized or insolubilizable antibodies useful in this embodiment can be supplied as part of a water-insoluble reagent comprising a particulate material to which the antibodies are attached. Such particles are preferably spherical in shape, but the structural and spatial configurations are not critical. Generally, the largest dimension of the particles is from 0.01 to 10 $\mu$m and preferably the particles have a diameter of from 1 to 3 $\mu$m. They can be prepared from any natural or synthetic material to which antibodies can be adsorbed or covalently attached.

Preferred particles have suitable reactive groups for covalent attachment of antibodies, either directly or indirectly. Indirect covalent attachment is meant to include attachment through specific binding reactions such as through avidin-biotin complexes. Direct covalent binding can be accomplished using surface reactive groups which can react with free amine or sulfhydryl groups of the antibodies. Such groups include, but are not limited to, carboxy, epoxy, aldehyde, active halo atoms, activated 2-substituted ethylsulfonyl and vinylsulfonyl groups. Carboxy, active halo, activated 2-substituted ethylsulfonyl and vinylsulfonyl groups are preferred. Particularly useful polymeric particles include those described in copending EP-A-0 323 692.

In a preferred embodiment of this invention, a sandwich assay for the simultaneous, indiscriminate detection of any of the microorganisms A. actinomycetemcomitans, B. gingivalis and B. intermedius comprises the steps of:

A. extracting one or more antigens from any of the microorganisms A. actinomycetemcomitans, B. gingivalis and B. intermedius present in a biological specimen,

B. contacting the one or more extracted antigens with a microporous membrane in a disposable test device, the membrane having affixed substantially on one surface thereof a water-insoluble reagent comprising polymeric particles having covalently attached thereto antibodies directed to at least one antigen from each of the microorganisms,
to form water-insoluble immunological complexes between the antibodies and the one or more extracted antigens present on the membrane,

C. contacting the water-insoluble complexes on the membrane with enzyme-labeled, water-soluble antibodies directed to at least one antigen from each of the microorganisms so as to form an enzyme-labeled, water-insoluble sandwich complex of both the labeled and water-insoluble antibodies with the antigens on the membrane,

D. washing uncomplexed materials through the membrane, and

E. contacting the enzyme-labeled sandwich complex on the membrane with a composition which provides a dye in the presence of the enzyme to visually indicate the presence of the one or more antigens as an indication of any of the microorganisms in the specimen.

The diagnostic test kit of this invention includes the article of this invention as well as a plurality of detectably labeled antibodies directed to the microorganisms of interest. These kit components can be packaged in a suitable manner and included in a carrier of some type which can be compartmentalized to hold the article (alone or in a test device) and vials or bottles of reagents. In addition, it can also include one or more of the following which are optionally useful in carrying out the screening method: dye-forming composition, extraction reagents, wash solutions, diluents, and other reagents known to one skilled in the art for a given assay format. Reagents can be provided in dry form or in appropriate solutions. Non-reactive components of the kit can include instructions, mixing vessels, stirring means, pipettes and the like.

The following preparation of polyclonal antibodies is a preferred method for their preparation.

The bacterial strains were supplied as viable cultures by Homer S. Reynolds (SUNY, Buffalo, School of Dentistry) Isolates were anaerobically cultured on CDC anaerobic plates (BBL Laboratories, Baltimore, Md.) supplemented with hemin (5 $\mu$g/ml) and menadione (0.5 $\mu$g/ml).

The plates were then incubated for 24-48 hours at 37°C in an anaerobic chamber. Frozen stocks were prepared for each strain by harvesting the colonies with an inoculating loop, preparing a cell suspension in skim milk and freezing the suspension at -70°C. Viability of the frozen stock was tested by plating a portion of the frozen stock onto CDC anaerobic plates and incubating as described above. Once viability was determined, fresh isolates for each strain could be obtained in a similar manner. Cell suspensions were prepared by harvesting colonies with an inoculating loop, placing the loop in phosphate buffered saline solution (pH 7.5, 1 ml) and vortexing vigorously for about one minute.

Concentrations of cell suspensions were determined spectrophotometrically by measuring turbidity at 620 nm. Appropriate dilutions were prepared to yield an optical density in the range of 0.1 to 1. Concentrations were determined using a 1 McFarland standard wherein $OD_{620}$ = 0.180, corresponds to about $3 \times 10^8$ cells/ml.

Ten New Zealand white rabbits were intravenously injected with 0.5 ml of a phosphate buffered saline solution (pH 7.3) solution of the respective immunogen containing about 5 x $10^8$ whole viable cells per ml. The injections were given following the method shown in McCarty and Lancefield (J.Exp.Med., 102, pp. 1-28, 1955).

Two booster injections were given as follows: one week after the initial injection, each rabbit was injected again with the same amount of immunogen, and one additional week later, each rabbit was similarly injected.

Beginning with the fifteenth day after the initial injection, for a total of eleven additional weeks, each rabbit was given a booster injection of 1 ml of immunogen (5 x $10^8$ cells/ml) three times per week. The booster injections were spaced out every other day over each seven day period. Samples (2 ml) of antisera (for each serotype) were collected from the rabbits at various times to determine the antibody performance at those times. For example, antisera samples were taken at three, six and nine week intervals after the initial injection of immunogen. The rabbits were then sacrificed at thirteen weeks after the initial injection and the antisera was collected (about 100 ml per rabbit).

The final sera were then purified using ammonium sulfate precipitation. Saturated ammonium sulfate solution was added dropwise with stirring to each serum sample, cooled on ice until 45% saturation was achieved. After the addition, the mixture was stirred for an additional ten minutes, centrifuged and the supernatant discarded. The pellet was resuspended in a volume of phosphate buffered saline solution (pH 7.3) equal to the amount of the original sample being purified. The noted steps (addition of ammonium sulfate, centrifugation and resuspension of the pellet) were repeated. The mixture was then transferred to dialysis bags and dialyzed for about fifteen hours at 4°C with stirring against phosphate buffered saline solution (pH 7.3). About 200-10,000 times excess of dialysis buffer was used. The dialysis was repeated with fresh buffer for an additional six hours. The solutions were removed from the dialysis bags and filtered through a 0.22 $\mu$meter filter. A portion of the filtrate was diluted in the range of 1:10 to 1:100 in phosphate buffered saline solution, and the absorbance was read at 280 nm. The antibody concentration was determined according to the formula:

$$A_{280} \div [1.4 \text{ x (dilution)}] = \text{antibody conc.(mg/ml)}$$

The antibody solutions were then diluted to 2-4 mg/ml with phosphate buffered saline solution (pH 7.3) and merthiolate (0.01 weight %) and stored at 4°C (for small amounts whereas large amounts were stored at -70°C).

The following examples are provided to illustrate this invention, but are not to be limiting in any way. All percentages are based on weight unless otherwise indicated.

Example 1: Direct Binding Screening Assay

This example illustrates the use of a direct binding assay to screen for any of the microorganisms A. actinomycetemcomitans, B. gingivalis and B. intermedius in a biological specimen.

Materials and Methods:

Surecell™ disposable test devices (Eastman Kodak Co.) were used, each containing three test wells having Biodyne™ A nylon microporous membranes (5 $\mu$m, Pall Corp.) in the bottom thereof. The membranes had been coated with Zonyl™ FSN nonionic surfactant (0.05 g/m², DuPont).

A blocking composition comprised casein (5%), merthiolate (0.01%) in 3-(N-morpholino)propanesulfonic acid buffer (0.01 molar, pH 7.5).

Polyclonal antibodies directed against A. actinomycetemcomitans (serotypes A, ATCC 43717 and B, ATCC 43718), B. gingivalis (serotypes A, ATCC 33277 B, ATCC 53978 and C, ATCC 53977) and B. intermedius (serotypes A, ATCC 25611 B, NCTC 9336 and C, ATCC 49046) were obtained by intravenous injection of rabbits using the general procedure described above. IgG fractions were prepared by ammonium sulfate precipitation and stored at 4°C in phosphate buffered saline solution (0.3-0.4%). Large amounts of antibodies were stored at -70°C.

The bacterial strains used to produce the antisera were supplied in viable cultures by H.S. Reynolds (School of Dentistry, SUNY, Buffalo). Isolates were subcultured as described above.

Enyzme-antibody conjugates, directed against each serotype, were prepared by covalently binding horseradish peroxidase to specific rabbit polyclonal antibodies by the procedure of Yoshitake and others (Eur. J. Biochem., 101, 395, 1979). The conjugate composition comprised 3 μg of each conjugate per ml of blocking composition.

Antigens from the bacterial strains, corresponding to the serotypes above, were extracted using phosphate buffered saline solution (pH 7.3) for about 1 minute at room temperature.

A wash solution comprised 3-cyclohexylamino-2-hydroxyl-propanesulfonic acid buffer (0.5 molar), Emcol™ CC-9 cationic surfactant (0.75%, Witco Chemical Co.) and merthiolate (0.01%). The pH of the solution was adjusted to 10 by the addition of sodium hydroxide (0.05 normal).

A dye-providing composition was prepared to include 2-(4-hydroxy-3,5-dimethoxyphenyl)-4,5-bis(4-methoxyphenyl)imidazole (0.008%), poly(vinylpyrrolidone) (1%), sodium phosphate buffer (10 mmolar, pH 6.8), hydrogen peroxide (10 mmolar), 4'-hydroxyacetanilide electron transfer agent (2 mmolar) and diethylenetriaminepentaacetic acid chelating agent (10 μmolar).

Assay:

The solution of extracted antigens (50 μl) was added to the wells of a test device and the antigens became bound to the membrane as the extraction solution flowed through. This took only a few minutes. Phosphate buffered saline solution (50 μl) was added to a test well as a negative control. The conjugate composition (50 μl) was then added to the wells, followed by incubation at room temperature for 5 minutes to allow complexation of antigen and antibodies.

The wash solution (240 μl) was added to the test wells twice, followed by addition of the dye-providing composition (50 μl). After incubation at room temperature for 2 minutes, the dye on the membrane was visually observed and compared to a calibrated color chart with reflectance density values, then converted to transmittance density ($D_T$) using the Williams-Clapper transform (J. Opt. Soc. Amer., 43, 595, 1953).

The results, shown in Table I below, indicate that the method of this invention can be used in a binding assay format to screen for all three microorganisms, A. actinomycetemcomitans, B. gingivalis and B. intermedius, in one test device.

## T A B L E   I

| Microorganism (Serotype) | Cell Concentration | $D_T$ |
|---|---|---|
| A. actinomycetemcomitans (A) | $3.3 \times 10^6$ | 0.215 |
| A. actinomycetemcomitans (A) | $1.95 \times 10^5$ | 0.195 |
| B. gingivalis (B) | $6.25 \times 10^6$ | 0.175 |
| B. gingivalis (B) | $7.81 \times 10^5$ | 0.101 |
| B. intermedius (A) | $6.25 \times 10^6$ | 0.215 |
| B. intermedius (A) | $7.81 \times 10^5$ | 0.175 |
| Control (phosphate buffered saline solution) | 0 | 0.015 |

Example 2: Sandwich Screening Assay

This example illustrates the use of a sandwich assay to screen for any of the microorganisms A. actinomycetemcomitans, B. gingivalis and B. intermedius in a biological specimen.

Materials and Methods:

Surecell ™ disposable test devices (Eastman Kodak Co.) were used in the tests. They contained three test wells each having a Loprodyne™ microporous membrane (5 μm, Pall Corp.) in the bottom thereof. Each membrane had been pretreated with Fluorad™ FC 135 nonionic surfactant (0.05 g/m², 3M).

Antibodies to A. actinomycetemcomitans [equal concentrations of serotypes A and B, ATCC 43717 and ATCC 43718, respectively, Zambon and others, Infect. Immun., 41(1), pp. 19-27, 1983] were covalently

EP 0 439 214 A1

bound to poly[styrene-co-m & p-(2-chloroethylsulfonylmethyl)styrene] (96:4 molar ratio) polymeric particles (1.5 -2.5 $\mu$m average diameter) using the method described in EP-A-0 323 692 (noted above) to provide a reagent having about 9 mg of antibody attached per g of polymer.

In the same manner, antibodies to B. intermedius (equal concentrations of serotype A, ATCC 25611, serotype B, NCTC 9336 and serotype C, ATCC 49046) were covalently bound to the same type of polymeric particles. Antibodies to B. gingivalis (equal concentrations of serotype A, ATCC 33277, serotype B, ATCC 53978 and serotype C, ATCC 53977) were covalently bound to similar polymeric particles.

Each polymer-antibody reagent thus formed was formulated into a composition containing the reagent (1%) and polyacrylamide (5%) in glycine buffer (0.1 molar, pH 8.5).

A conjugate composition was prepared as described in Example 1 above.

Strains of the microorganisms were supplied as viable cultures by H.S. Reynolds (SUNY, Buffalo School of Dentistry), and isolates were obtained as described above.

The culture suspensions were extracted with sodium dodecyl sulfate (10% solution) for about 1 minute at room temperature.

The wash solution comprised sodium decyl sulfate (18 g/l).

The dye-providing composition was like that used in Example 1.

Assay:

Equal volumes of the particle-antibody reagent compositions were mixed together and applied (3 $\mu$l) to defined regions of membranes in the test devices. To one set of wells was added various concentrations (50 $\mu$l) of extracted antigen of A. actinomycetemcomitans, followed by addition of the conjugate composition (50 $\mu$l) To a second set of test wells was added various concentrations (50 $\mu$l) of extracted B. intermedius antigens, followed by addition of the conjugate composition (50 $\mu$l) To a third set of test wells, was added various concentrations (50 $\mu$l) of extracted B. gingivalis antigens, followed by addition of the conjugate composition (50 $\mu$l) A negative control was situated as the area around the defined regions on the membranes where the particle-antibody reagents had been applied.

All of the test wells were incubated for 5 minutes at room temperature, and washed twice with the wash solution (240 $\mu$l). The dye-providing composition (50 $\mu$l) was then added, followed by incubation at room temperature for 2 minutes. The observed dye in each test was compared to a calibrated color chart of reflectance density, then converted to transmittance density ($D_T$) as described in Example 1.

The results, shown in Table II below indicate that the detection of each microbial species can be achieved using a single test device.

## T A B L E   II

| Antigen Level (# of Cells) | $D_T$ Actinobacillus Actinomycetem-comitans | Bacteroides intermedius | Bacteroides gingivalis |
|---|---|---|---|
| $6.25 \times 10^6$ | 0.215 | 0.101 | 0.175 |
| $7.81 \times 10^5$ | 0.175 | 0.042 | 0.114 |
| $9.75 \times 10^4$ | 0.101 | 0.015 | 0.101 |
| Negative Control (no cells) | 0.003 | 0.003 | 0.003 |

## Claims

1. A method for the simultaneous, indiscriminate detection of any microorganism associated with periodontal disease comprising:

A. contacting a specimen suspected of containing one or more antigens extracted from one or more microorganisms associated with periodontal disease with a microporous substrate, and forming on

9

the substrate one or more immunological complexes by contacting the one or more antigens simultaneously with one or more antibodies directed to a plurality of microorganisms associated with periodontal disease, and

B. detecting the presence of the one or more complexes on the substrate as an indication of the presence of at least one of the microbial antigens in the specimen.

2. The method as claimed in claim 1 wherein the one or more antigens are directly bound to the substrate, and the microbial antibodies complex with the antigens to form immunological complexes bound directly to the substrate.

3. The method as claimed in claim 2 wherein the microbial antibodies are unlabeled, and the complexes are subsequently contacted with labeled antibodies directed against each of the microbial antibodies to form detectable antibody-antibody-antigen complexes bound to the membrane.

4. The method as claimed in either of claims 1 and 2 wherein the microbial antibodies are labeled for detection of the immunological complexes.

5. The method of claim 1 wherein the antibodies are randomly affixed to the substrate, and prior to, simultaneously with or subsequently to the contact of the affixed antibodies and the one or more antigens, contacting the one or more antigens with water-soluble second antibodies directed to at least one antigen from each of the microorganisms, to form a sandwich complex of the one or more antigens with the affixed and second antibodies, the second antibodies being labeled for detection.

6. The method as claimed in claim 5 wherein the affixed antibodies are covalently bound to polymeric particles applied to the substrate.

7. The method as claimed in either of claims 5 and 6 wherein the substrate is a microporous membrane and the antibodies are affixed to a discrete zone which is less than the total surface area of the substrate.

8. The method as claimed in any of claims 1 to 7 for the simultaneous, indiscriminate detection of Actinobacillus actinomycetemcomitans, Bacteroides gingivalis and Bacteroides intermedius.

9. A water-insoluble article comprising a microporous substrate having first and second opposing outer surfaces,

the article characterized wherein randomly affixed to at least one of the surfaces are one or more antibodies directed to a plurality of microorganisms associated with periodontal disease, the antibodies being substantially all on the first or second surface.

10. The article as claimed in claim 9 wherein the one or more antibodies are affixed to a discrete zone which is less than the total area of the first or second surface of the substrate.

11. The article as claimed in either of claims 9 or 10 wherein the one or more antibodies are covalently bound to polymeric particles as part of a water-insoluble reagent which is located on the substrate.

12. The article as claimed in any of claims 9 to 11 wherein less than 1%, by weight, of the one or more antibodies are entrapped by the membrane.

13. A test kit for the simultaneous, indiscriminate detection of any microorganism associated with periodontal disease comprising:

a. a water-insoluble article comprising a microporous substrate having first and second opposing outer surfaces, and one or more unlabeled antibodies randomly affixed to at least one of the surfaces, and

b. detectably labeled antibodies having the same reactivity as the affixed antibodies, the labeled antibodies being packaged separately or in admixture,

the kit characterized wherein the unlabeled antibodies are directed to a plurality of microorganisms associated with periodontal disease, and the unlabeled antibodies are substantially all on the first or second surface.

European
Patent Office

EUROPEAN SEARCH
REPORT

Application Number

EP 91 20 0049

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | EP-A-0 269 388 (MINNESOTA MINING AND MANUFAC-TURING CO.)<br>* Page 3, lines 63-64; page 13, examples 6,7; page 15, example 9 * | 1-13 | G<br>01 N 33/569<br>G 01 N 33/543<br>G 01 N 33/546 |
| Y | EP-A-0 253 464 (HYBRITECH INC.)<br>* Page 8, lines 22-29; figure 3 * | 1-13 | |
| T | JOURNAL OF DENTAL RESEARCH (US), vol. 69, no. SPEC, March 1990, page 243, abstract no. 1077, US; P. CONTESTABLE et al.: "A rapid enzyme immunoassay for three suspected periodontal pathogens"<br>* Whole abstract * | 1-13 | |
| A | EP-A-0 335 244 (ABBOTT LABORATORIES)<br>* Page 5, line 1 - page 6, line 40; page 8, lines 20-37 * | 6,11-13 | |

TECHNICAL FIELDS
SEARCHED (Int. Cl.5)

G 01 N

The present search report has been drawn up for all claims

| Place of search | Date of completion of search | Examiner |
|---|---|---|
| The Hague | 19 April 91 | VAN BOHEMEN C.G. |